Europäisches Patentamt

European Patent Office

Office européen des brevets

(11) Publication number: **0 492 530 A1**

## EUROPEAN PATENT APPLICATION

(21) Application number: **91121998.8**

(22) Date of filing: **20.12.91**

(51) Int. Cl.5: **C12N 5/20**, C12P 21/08,
G01N 33/577, G01N 33/68,
A61K 47/48

The microorganism(s) has (have) been deposited with American Type Culture Collection under number(s) ATCC HB 10433.

(30) Priority: **24.12.90 US 632705**

(43) Date of publication of application:
**01.07.92 Bulletin 92/27**

(84) Designated Contracting States:
**AT BE CH DE DK ES FR GB GR IT LI LU NL SE**

(71) Applicant: **E.R. SOUIBB & SONS, INC.**
**P.O.Box 4000**
**Princeton New Jersey 08543-4000(US)**

(72) Inventor: **Eager, Kendra B.**
**702 Windsor Commons**
**Cranbury, NJ(US)**

(74) Representative: **Vossius & Partner**
**Siebertstrasse 4 P.O. Box 86 07 67**
**W-8000 München 86(DE)**

(54) Monoclonal antibodies which bind mannose binding protein.

(57) Monoclonal antibodies which bind mannose binding protein, hybrid cell lines which produce these monoclonal antibodies, and immunoassay methods for detecting mannose binding protein using these monoclonal antibodies.

EP 0 492 530 A1

The fusion of mouse myeloma cells to spleen cells derived from immunized mice by Köhler and Milstein in 1975 [Nature 256, 495-497 (1975)] demonstrated, for the first time, that it was possible to obtain continuous cell lines making homogeneous (so-called "monoclonal") antibodies. Since this seminal work, much effort has been directed to the production of various hybrid cell lines (also called "hybridomas") and to the use of the antibodies made by these hybridomas for various scientific investigations. While the general technique for the preparation of hybridomas and monoclonal antibodies is well-known, there are many difficulties met and variations required for each specific case. In fact, there is no assurance, prior to attempting to prepare a given hybridoma, that the desired hybridoma will be obtained, that it will produce antibody if obtained, or that the antibody so produced will have the desired specificity.

Mannose binding proteins (MBP) have been isolated from a number of different mammalian species, including rabbits, rodent and humans. These proteins have a high affinity for mannan and mannose oligosaccharides which are present in the cell wall of many pathogens, including some gram-negative bacteria, mycobacteria, yeast, fungi and in the envelope glycoproteins of viruses such as the human immunodeficiency virus (HIV). The major human serum mannose binding protein is a polymer of approximately 300 kDA comprised of subunits of 32 kDa, is calcium dependent and is produced by the liver.

The pattern of expression of this protein as well as the examination of the promotor region of the gene indicate that this is an acute phase reactant protein. Several investigators have shown that these proteins bind to yeast mannans and interact with the complement cascade [(Kawasaki, M., et al., J. Biochem (Tokyo) 94, 937 (1983); Ikeda, K. et al., J. Biol. Chem. 262, 7451 (1987)]. It has been suggested from these findings that the proteins may play a role in host defenses by inactivating those organisms bearing the mannan residues. Low levels of MBP in patients has been correlated with a defect in the ability of opsonization [Super, M. et al., Lancet ii, 1236-1239 (1989)]. The median level of MBP in affected children measured 4.9 $\mu$g/l compared with 143 $\mu$g/l in the pediatric control group. It has been demonstrated that mannose binding protein can inhibit HIV infection *in vitro* and also binds selectively to HIV-infected cells [(Ezekowitz, R.A.B. et al., J. Exp. Med. 169, 185-196 (1989)].

Thus it is readily apparent that the need exists in the art for materials and methods for the study of mannose binding proteins so that its role in host defense mechanisms may be further elucidated. There are also needs for methods for quantitating levels of mannose binding proteins so that appropriate therapy can be provided.

The present invention concerns hybrid cell lines which produce monoclonal antibodies which bind mannose binding protein.

The present invention further concerns monoclonal antibodies which bind mannose binding protein.

The present invention additionally concerns immunoassay methods for detecting the presence of mannose binding protein in a sample.

The present invention also concerns immunoassay methods for quantitatively determining the amount of mannose binding protein in a sample.

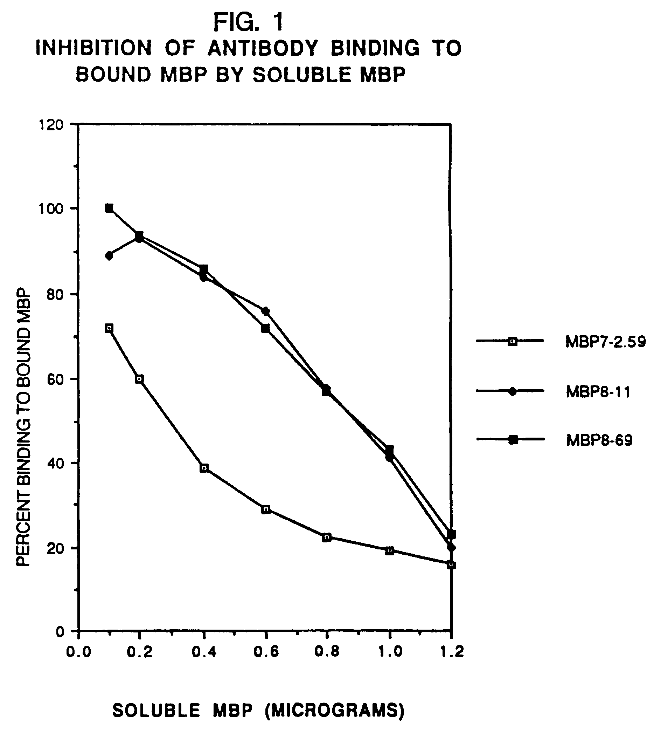

Figure 1 shows the inhibition of antibody binding to bound mannose binding protein by soluble mannose binding protein.

The present invention concerns hybrid cell lines, also called hybridomas, monoclonal antibodies and immunoassay methods utilizing these antibodies.

In particular, the present invention concerns hybrid cell lines which produce monoclonal antibodies which bind mannose binding protein.

Particularly preferred is the hybrid cell line designated as MPB7-2.59, which is a subclone of MBP7-2, or hybrid cell lines which have the identifying characteristics of this hybrid cell line.

Hybrid cell line MBP7-2.59 was deposited with the American Type Culture Collection, Rockville, Maryland on April 19, 1990 under the Budapest Treaty and assigned ATCC accession no. HB 10433.

Also preferred are the hybrid cell lines designated as MBP8-11, MBP8-69 and MBP13-86, or subclones derived therefrom, or hybrid cell lines which have the identifying characteristics of these hybrid cell lines.

The hybrid cell lines of the present invention may be produced by various methods generally known to those of ordinary skill in the art. In general, the method involves immunizing suitable mammals, for example mice, with the antigen of interest, in this case mannose binding protein, fusing antibody producing cells isolated from the animal with myeloma cells, cloning the resulting hybrid cells and selecting those cells which produce the desired monoclonal antibody which binds the antigen of interest.

Immunizations are usually performed with purified antigens, in this case purified mannose binding protein.

The usual mammals used for immunizations are mice, especially BALB/c mice, but other mammals and mouse strains may also be employed. The immunizations are performed in a manner known in the art, such as by administering parenterally, intraperitoneally, intravenously and/or subcutaneously, three to six injections each containing an appropriate amount of purified antigen (i.e, from about 1 $\mu$g to about 50 $\mu$g), at intervals of about one to six weeks, usually together with an adjuvant, for example, complete or incomplete Freund's adjuvant. While immunizations are generally performed in vivo, various in vitro procedures are also known and may be employed.

Antibody-producing cells of the immunized animals, usually spleen cells, are taken from the animals two to six days after the last ("booster") immunization and fused with myeloma cells of a suitable cell line. Myeloma cell lines and cell lines derived therefrom are known as suitable fusion partners. The myeloma cell line is generally derived from the same species as the immunized mammal, since intra-species hybrids are more viable than inter-species hybrids. Myeloma cells that lack the enzyme hypoxanthine-guaninephosphoribosyl transferase (HGPRT) or the enzyme thymidine kinase (TK) and that, for that reason, do not survive in a selective culture medium containing hypoxanthine, aminopterin and thymidine (HAT medium), may be employed. Myeloma cells and cell lines prepared therefrom that do not survive in HAT medium and do not secrete any immunoglobulins or parts thereof, for example cell lines P3X63-Ag8.653 and Sp2/0-Ag14, may also be used. Various fusion-promoters, for example, Sendai virus or other paramyxoviruses, optionally in UV-inactivated form, calcium ion, surface-active lipids, such as isolecithin, or polyethylene glycol may also be employed. Myeloma cells are usually fused with a three- to twenty-fold excess of spleen cells from immunized animals in a solution containing from 30 to 50% polyethylene glycol (PEG) having a molecular weight of about 1000 to 4000. Exposure to PEG for about 2 to 3 minutes appears to be optimal to prevent toxicity to cells; temperatures of about 37° are recommended.

After fusion, the cells are partitioned out and cultured in selective HAT medium, with only hybrid cells surviving, since these combine, from the myeloma cells, the ability to grow in vitro and, from the antibody-producing cells of the immunized animals, the missing HGPRT or TK genes and, therewith, the ability to survive in HAT medium.

Suitable culture media for the growth of the hybridoma cells are the customary standard culture media, for example, Dulbecco's Modified Eagles Medium or Roswell Park Memorial Institute (RPMI) 1640 medium containing 10-15% fetal calf serum, supplemented with antibiotics. At the beginning of cell growth, so-called feeder cells, for example normal mouse peritoneal exudate cells, spleen cells, bone marrow macrophages or the like, may be added. At regular intervals, said culture media may be supplemented by selective HAT medium to prevent hybrid cells from being overgrown by ordinary myeloma cells still present after the initial HAT selection process.

The cell culture supernatants of the hybrid cells surviving HAT selection are examined for the presence of the desired monoclonal antibodies. Advantageously, the cell supernatants are tested in an immunoassay, for example, radioimmunoassay or enzyme immunoassay, that demonstrates the binding of monoclonal antibodies to the antigen of interest.

Those hybrid cells which produce antibodies having the desired specificity as well as other desirable characteristics can then be maintained as viable cultures and/or frozen for storage.

The present invention further concerns monoclonal antibodies which bind mannose binding protein.

Preferred are the monoclonal antibodies designated as MabMBP7-2, MabMBP8-11, MabMBP8-69 and MabMBP13-86, or monoclonal antibodies with the identifying characteristics of these monoclonal antibodies.

Particularly preferred is the monoclonal antibody designated as MabMBP7-2.59, or monoclonal antibodies with the identifying characteristics of this monoclonal antibody.

Also preferred are substantially purified monoclonal antibodies which bind mannose binding protein.

Additionally preferred are derivatives of monoclonal antibodies which bind mannose binding protein.

The monoclonal antibodies of the present invention may be produced by various methods generally known to those of ordinary skill in the art. Hybrid cells producing such antibodies may be cultured in vitro and the monoclonal antibodies isolated from the culture supernatants, or may be multiplied in vivo in a suitable mammal, and the monoclonal antibodies isolated from the body fluids of that mammal. If desired, a monoclonal antibody resulting from either of these techniques may be converted into a derivative thereof.

Suitable culture media for in vitro culturing are the customary standard culture media, for example, Dulbecco's Modified Eagles Medium or RPMI 1640 medium containing 10 to 15% fetal calf serum and supplemented with antibiotics.

Large quantities of the desired monoclonal antibodies may also be obtained by multiplying the hybrid cells in vivo. For this purpose, antibody producing hybridomas are inoculated intraperitoneally into syngeneic mammals, and after 1 to 3 weeks, the antibodies are isolated from the ascites fluid of those mammals. For example, hybrid cells originating from BALB/c mice are injected intraperitoneally into BALB/c mice that have previously been pretreated intraperitoneally with a hydrocarbon such as 2,6, 10,14-tetramethylpentadecane (pristane) to irritate the peritoneal cavity, and, after 8 to 10 days, ascites fluid is withdrawn from these animals.

The monoclonal antibodies produced in vitro or in vivo may be purified using various methods, for example, gel filtration chromatography, ion-exchange chromatography, DEAE-cellulose chromatography or affinity chromatography. Optionally, selected proteins in the culture supernatants or ascites fluid, including the desired monoclonal antibodies, may be precipitated using specific concentrations of ammonium sulphate or the like before being subjected to chromatography.

If desired, derivatives of the monoclonal antibodies produced either in vitro or in vivo may be prepared.

Derivatives of monoclonal antibodies according to the invention include, for example, fragments, such as Fab, Fab' or F(ab')$_2$ fragments, that retain their specificity for the antigenic determinants of the antigen of interest, radioactively labelled monoclonal antibodies which are labelled, for example, with radioactive iodine ($^{125}$I, $^{131}$I), carbon ($^{14}$C), sulphur ($^{35}$S), tritium ($^3$H) or the like, and monoclonal antibodies conjugated with enzymes such as horseradish peroxidase, alkaline phosphatase, $\beta$-D-galactosidase, glucose oxidase, glucoamylase, carbonic anhydrase, acetylcholinesterase, lysozyme, malate dehydrogenase or glucose-6-phosphate dehydrogenase. Additional derivatives include monoclonal antibodies labeled with fluorescent materials such as fluorescein or rhodamine, and monoclonal antibodies labelled with biotin.

Fragments of monoclonal antibodies according to the invention, for example, Fab, Fab' or F(ab')$_2$ fragments, that retain their specificity for the antigenic determinants of the antigen of interest, may be prepared according to generally known methods, for example, by fragmenting monoclonal antibodies by proteolytic digestion with enzymes such as pepsin or papain and/or by cleavage of disulphide bonds by chemical reduction.

Monoclonal antibodies radioactively labelled with iodine ($^{125}$I, $^{131}$I) may be obtained by iodination, for example, with radioactive sodium or potassium iodide after oxidization with a chemical oxidizing agent, such as sodium hypochlorite, chloramine T or the like, or an enzymatic oxidizing agent, such as lactoperoxidase and glucose oxidase. Radioactively labelled monoclonal antibodies according to the invention may also be prepared by adding, to the culture media for the in vitro culturing, in a known manner, radioactively labelled nutrients containing radioactive carbon ($^{14}$C), tritium ($^3$H), sulphur ($^{35}$S) or the like, for example, L-($^{14}$C)-leucine, L-($^3$H)-leucine or L-($^{35}$S)-methionine, and obtaining the monoclonal antibodies as described above.

Enzyme-conjugated monoclonal antibodies according to the invention may be obtained by various generally known methods, for example, by reacting monoclonal antibodies and the desired enzyme after modification with coupling reagents such as aldehydes, carbodiimides, maleimides, imidates, succinimides and pyridyl disulfides. Specific coupling agents include, for example, glutaraldehyde, periodate, N,N'-o-phenylenedimaleimide, N-(m-maleimidobenzoyloxy)-succinimide, N-(3-(2'-pyridyldithio)-propionoxy)-succinimide or the like.

Various enzyme substrates, for example 5-aminosalicyclic acid, O-phenylenediamine, 3,3'-dimethoxybenzidine, and 2,2'-azino-bis-(3)-ethylbenzothiazolin-6-sulphonic acid for horseradish peroxidase and p-nitrophenyl phosphate for alkaline phosphatase, may be used in conjunction with the enzyme-conjugated antibodies.

Other derivatives contemplated by the present invention include, for example, monoclonal antibodies conjugated to a toxin, such as diptheria toxin, ricin or deglycosylated ricin A chain, or some other substance (e.g., an enzyme inhibitor such as glucosidase inhibitor) for therapeutic purposes. Such derivatives may be prepared using methods generally known in the art. Such derivatives can be used to treat infections caused by pathogens which contain mannan and mannose oligosaccharides in their cell walls (e.g., some gram-negative bacteria, mycobacteria, yeasts and fungi), or in the case of viruses such as the HIV virus, in their envelope glycoproteins. In using these monoclonal antibody derivatives to treat infections caused by pathogens, the monoclonal antibody derivatives may be administered in an appropriate manner (e.g., using a parenteral solution) to various mammalian species known to be subject to such infections, e.g., humans, cats, dogs and the like, in a therapeutically effective amount within the dosage range of about 0.01 to 100 mg/kg/day, preferably about 0.1 to 10 mg/kg/day, on a regimen in single or 2 to 4 divided daily doses. Alternatively, the solution may be continuously administered to supply these dosage amounts. The active substance should be utilized in a solution containing about 1.0 to about 10.0 mg per unit of dosage of the derivatized monoclonal antibody. They may be formulated in a conventional manner along with other physiologically acceptable materials, such as preservatives and stabilizers, as called for by accepted pharmaceutical practice.

If desired, in order to reduce antigenicity in humans, genomic DNA fragments encoding the heavy and light chain variable regions of the mouse monoclonal antibodies which bind mannose binding protein can be cloned and transferred into expression vectors that can result in the production of human immunoglobulins that retain the specificity of the mouse antibody as described in Sun, L. K. et al., Proc. Natl. Acad. Sci. (USA) 84, 214-218 (1987).

It should be understood that fragments of the monoclonal antibodies of the present invention may also be labeled with the various materials referred to above, for example, radioisotopes, toxins, enzymes, fluorescent materials and biotin, in the same manner described above for the full length monoclonal antibody molecules. Such variations are included within the scope of the present invention.

It is contemplated that the present invention encompasses all monoclonal antibodies exhibiting the characteristics of the monoclonal antibodies described herein. The monoclonal antibodies described herein belong to the classes IgG and IgM and the subclass $IgG_1$. It is contemplated that antibodies having the patterns of reactivity illustrated herein are included within the scope of the present invention regardless of the immune globulin class or subclass to which they belong. For example, a monoclonal antibody exhibiting the characteristics described herein may be of the subclass $IgG_1$, $IgG_2a$, $IgG_2b$, or $IgG_3$, or of classes IgM, IgA, or of other known Ig classes.

Furthermore, since the hybrid cell line produced from a known mouse myeloma cell line and spleen cells from a known species of immunized mouse cannot be further identified except by reference to the antibody produced by the hybrid cell line, it is contemplated that all hybrid cell lines producing antibodies having the reactivity characteristics described above are included within the scope of the present invention.

The present invention further concerns immunoassay methods utilizing monoclonal antibodies and derivatives thereof which bind mannose binding protein for the qualitative and quantitative determination of mannose binding protein, especially in a biological sample.

Particularly preferred is a qualitative immunoassay method for detecting the presence of mannose binding protein in a sample comprising:

(a) incubating the sample with a monoclonal antibody which binds to the mannose binding protein; and
(b) detecting the presence of immune complexes formed by the mannose binding protein and the monoclonal antibody.

Additionally preferred is an immunoassay method for quantitatively determining the amount of mannose binding protein in a sample comprising:

(a) incubating the sample with a monoclonal antibody which binds to the mannose binding protein;
(b) determining the amount of immune complexes formed by the mannose binding protein and the monoclonal antibody;
and
(c) correlating the amount of immune complexes formed with the amount of mannose binding protein present in the sample.

The immunoassay method of the present invention may be a radioimmunoassay (RIA) which utilizes, depending on the particular protocol employed, unlabeled or radioactively labeled derivatives of monoclonal antibodies which bind mannose binding protein, either alone or in combination. In the case where the monoclonal antibody which binds mannose binding protein is unlabeled, a different detectable marker, for example, a radiolabeled antibody which is capable of binding the monoclonal antibody which binds mannose binding protein, may be employed. Any of the known modifications of RIA, for example, homogeneous RIA, heterogeneous RIA, competitive RIA, and sandwich RIA, may be employed. Also contemplated are immunoblotting immunoassay techniques such as Western blotting employing a radioactive detection system.

The immunoassay method of the present invention may also be an enzyme immunoassay (EIA) which utilizes, depending on the particular protocol employed, unlabeled or enzyme-labeled derivatives of monoclonal antibodies which bind mannose binding protein, either alone or in combination. In the case where the monoclonal antibody which binds mannose binding protein is not enzyme labelled, a different detectable marker, for example, an enzyme-labeled antibody capable of binding to the monoclonal antibody which binds mannose binding protein, may be employed. Any of the known modifications of EIA, for example, enzyme-linked immunoabsorbent assay (ELISA) and sandwich EIA, may be employed. Also contemplated by the present invention are immunoblotting immunoassay techniques such as western blotting employing an enzymatic detection system.

The immunoassay method of the present invention may also be other known immunoassay methods, for example, fluorescent immunoassays using antibody conjugates or antigen conjugates of fluorescent substances such as fluorescein or rhodamine, latex agglutination with antibody-coated or antigen-coated latex particles, haemagglutination with antibody-coated or antigen-coated red blood corpuscles, and immunoassays employing an avidin-biotin or strepavidin-biotin detection system.

The particular parameters employed in the immunoassays of the present invention can vary widely depending on various factors such as the concentration of antigen in the sample, the nature of the sample, the type of immunoassay employed and the like. Optimal conditions can be readily established by those of ordinary skill in the art. The amount of antibody which binds mannose binding protein is typically selected to give 50% binding of detectable marker in the absence of sample. If purified antibody is used as the antibody source, the amount of antibody used per assay will generally range from about 1 ng to about 100 ng. Typical assay conditions include a temperature range of about 4°C to about 45°C, preferably about 25°C, a pH value range of about 5 to 9, preferably about 7, and an ionic strength varying from that of distilled water to that of about 0.2 M sodium chloride, preferably about that of 0.15 M sodium chloride. Times will vary widely depending upon the nature of the assay, and generally range from about 0.1 minute to about 24 hours. A wide variety of buffers, for example PBS, may be employed, and other reagents such as salt to enhance ionic strength, proteins such as serum albumins, stabilizers, biocides and non-ionic detergents may also be included.

The immunoassay methods of the present invention may be used in the identification and/or quantitation of mannose binding protein in the patient being tested. The presence of mannose binding protein in patients indicates infection, and has been correlated with a defect in oposonization.

It should be understood that fragments of the monoclonal antibodies of the present invention may be utilized in the immunoassay methods of the present invention, and that such variations are included within the scope of the present invention.

The monoclonal antibodies of the present invention may also be used to purify mannose binding protein. Briefly, monoclonal antibodies which bind mannose binding protein may be bound to a substrate (e.g., a solid support such as Protein-A Sepharose), and contacted with a material (e.g., a solution) containing mannose binding protein under conditions permitting the monoclonal antibodies to bind the mannose binding protein. Any unbound material is separated from the immobilized monoclonal antibodies, and the bound mannose binding protein eluted from the monoclonal antibodies with a suitable eluant to yield purified mannose binding protein.

The purified mannose binding protein may then be used therapeutically, as described in International Publication Number WO 89/01519.

Additional uses of the monoclonal antibodies which bind mannose binding protein, other than those described above, involve therapeutic uses. For example, the monoclonal antibodies can be linked to glucosidase inhibitor so that the antibody binds to mannose binding protein bound to a virus, thereby selectively delivering the enzyme inhibitor. The monoclonal antibodies can also be linked to a toxin to selectively kill a particular pathogen. In addition, the monoclonal antibodies which bind mannose binding protein can be used to potentiate the properties of mannose binding protein by a) alteration of the structure of the mannose binding protein-pathogen complex thus increasing the half life of the complex, or b) changing the conformation permitting increased or enhanced binding of mannose binding protein to the pathogen. These properties could be used in the delivery of natural or synthetic products (i.e. cytokines) to aid in immune response.

The following examples are further illustrative of the present invention. These examples are not intended to limit the scope of the present invention and provide further understanding of the invention.

Example 1

Cell Culture

The mouse myeloma (plasmacytoma) cell line, Sp2/0-Ag14, deficient in hypoxanthine guanine phosphoribosyl transferase (HGPRT) was employed. This cell line is available from the American Type Culture Collection (Rockville, MD) and the National Institute of General Medical Sciences Human Genetic Mutant Cells Depository (Camden, NJ). Sp2/0 cells and selected hybridomas were cultured at 37°C in a humidified 8% $CO_2$ atmosphere in Dulbecco's Modified Eagles Medium (DMEM) with high glucose (4.5 g/liter) supplemented with 10% fetal calf serum and L-glutamine (0.3 mg/ml). DMEM and L-glutamine were obtained from Gibco Laboratories (Life Technologies Inc., Grand Island, NY) and fetal calf serum was obtained from Hyclone Laboratories Inc., Logan, Utah. All other medium constituents were obtained from Sigma Chemical Company, St. Louis, MO unless otherwise indicated. After the fusion, cells were grown in HY medium (HY medium : DMEM supplemented with 10% NCTC 109 (Gibco), 15% fetal calf serum, 0.2 units bovine insulin/ml, 0.45 mM pyruvate, 1 mM oxaloacetate and 0.1% glutamine) containing hypoxanthine (0.1 mM), aminopterin (0.1 $\mu$m) and thymidine (0.016 mM).

Example 2

Production of Monoclonal Antibodies

Immunizations were performed with purified human mannose binding protein (immunogen) isolated from human sera by passage over a mannose-sepharose column [See, Ezekowitz, R.A.B. et al., J. Exp. Med. 169, 185-196 (1989)]. The purity of this protein was verified by polyacrylamide gel electrophoresis and immunoblotting.

BALB/c mice were hyperimmunized with doses of 15-25 $\mu$g of immunogen at 3-4 week intervals. The immunogen was emulsified in complete Freund's adjuvant for the first immunization and emulsified in incomplete Freund's adjuvant for the second immunization. The routes of immunization for these doses was subcutaneous and intraperitoneal. All subsequent immunizations were completed in saline (150 mM NaCl) and injected intraperitoneally. Mice were immunized at 3-4 week intervals, bled and the resultant sera analyzed for the presence of antibodies recognizing the immunogen by Western blotting methods. Mice having the highest serum titers were selected for fusions. Mice selected for fusion were rested for approximately one month and then immunized with the purified immunogen in saline for the three days prior to performing the fusion.

Fusions were performed according to a modification of the method of Köhler and Milstein Nature, 256, 495-497 (1975), using Koch-light polyethylene glycol 4000. Spleen cells from the selected animals were collected by perfusion with medium introduced by a syringe and erythrocytes lysed in cold 0.17 M $NH_4Cl$. The collected cells were counted and mixed at a ratio of $10^8$ spleen cells to $2 \times 10^7$ myeloma cells in a round-bottomed tube. The cell mixture was washed in medium free of serum by centrifugation. All supernatant liquid was removed by suction and the pellet loosened. 0.5 ml of PEG solution (30% polyethylene glycol, 5% dimethylsulfoxide in medium without serum) was slowly added to the pellet. The cells were maintained in the PEG solution for 8 minutes during which time they were pelleted at 1000 rpm for 5-6 minutes. Medium without serum (5 ml ) was slowly added to disperse the pellet followed by the addition of 5 ml of HY medium containing 15% fetal calf serum. The cells were pelleted and evenly resuspended in HY medium supplemented with hypoxanthine, aminopterin and thymidine resulting in a cell suspension of $1.5 \times 10^6$ cells per ml. Cells were then plated out in 96-well microtiter plates (100 $\mu$l/well) and placed in a humidified $CO_2$ incubator at 37°C. The wells were refed 6-7 days later. Clones growing in selection medium in microtiter plate wells were identified by examining the plate macroscopically using an inverted mirror stand. Medium from wells containing these clones was tested for the presence of specific antibody by ELISA.

Example 3

Expansion of Antibody Producing Hybridomas

Hybridomas producing specific antibody as demonstrated by ELISA were expanded by standard cell culture techniques and grown for several passages in HY media supplemented with hypoxanthine and thymidine. Cells were adapted to Dulbecco's Modified Eagles medium supplemented with 10% calf serum and glutamine (0.1%). Hybridomas of interest were subcloned by limiting dilution in freshly prepared HY media; clones were screened by ELISA. Subcloned hybridomas and the original lines were cryopreserved by standard techniques using a freezing mixture of 95% calf serum with 5% dimethylsulfoxide.

Antibody was collected in cell culture supernatant by accumulating antibody from densely growing cultures. In addition to cell culture methods, hybridomas were also grown in the peritoneal cavity of BALB/c mice. Mice were injected intraperitoneally with 0.5 ml of pristane (2,6,10,14-tetramethyldecanoic acid, Aldrich Chemical Company, Inc., Milwaukee, WI) at least 10 days prior to injection with the hybridoma line of interest. Hybridomas (approx $4 \times 10^6$ cells) shown to have activity in an ELISA prior to injection were inoculated intraperitoneally into the pristane treated BALB/c mice to produce ascites fluid. Ascites fluid was removed from the mice after 7-10 days and clarified by centrifugation at 35000 rpm in a Beckman 50 Ti rotor at 6°C. Clarified ascites was stored at 4°C with 0.02% sodium aside or frozen in aliquots at -70°C.

Example 4

Enzyme Linked Immunosorbent Assay (ELISA)

Sera from immunized mice and media collected from wells containing HAT selected hybridomas were tested for the presence of antibodies recognizing human mannose binding protein by three ELISA methods.

Method one consisted of purified mannose binding protein diluted in 0.05 M sodium carbonate buffer (pH 9.76) to 40 $\mu$g/ml adsorbed to polystyrene plates. 50 $\mu$l of this solution was added per well of a distilled water-rinsed Nunc Immuno Plate IF 96-well plate (A/S Nunc, Kamstrup, Denmark) and sealed. After incubation overnight at 4°C, the plates were rinsed six times with PBS-Tw20 (Phosphate buffered saline with 0.05% Tween 20). Undiluted media (50 $\mu$l) or diluted mouse sera (50 $\mu$l) were incubated for two hours at room temperature. Unbound mouse antibodies were removed by washing the plates six times with the PBS-Tw20 wash. After washing, 100 $\mu$l of diluted horseradish peroxidase conjugated-goat anti-mouse immunoglobulin IgG (heavy and light chain) (Organon Teknika-Cappel, Malvern, PA) was added per well for a period of two hours at room temperature. Antisera was diluted to 1:8000 in ELISA buffer consisting of PBS-Tw20 supplemented with 20.4 g sodium chloride per liter, 0.29 g EDTA per liter and 0.2% peroxidase-free bovine serum albumin. Removal of unbound conjugate was accomplished by extensive washings with PBS-Tw20. Bound conjugate was detected by visualization using a TMB (3,3',5,5' tetramethylbenzidine) substrate kit (TMB Microwell Peroxidase Substrate System; Kirkegaard and Perry, Gaithersburg, MD). Equal volumes of the TMB Peroxidase Substrate Solution and Peroxidase Substrate Solution B with hydrogen peroxide were mixed immediately prior to addition to the wells of the 96-well plate. 100 $\mu$l of the substrate solution was added per well and the reaction allowed to proceed for 15 minutes. The reaction products measured by optical densities were recorded on a Titertek Multiskan PLUS microtiter plate reader (Flow Laboratories, Inc., McLean, VA) at 650 nm. The reactions were stopped by the addition of 50 $\mu$l of 1 M HCl and the reactions read at 450 nm.

Method two consisted of the binding of monoclonal antibodies to mannose binding protein immobilized via a mannan residue attached to the polystyrene plate. Mannan (Sigma Chemical Co., St. Louis, MO) was prepared in carbonate buffer as described above and plated at a concentration of 0.4 micrograms per microtiter well. Mannose binding protein was diluted in carbonate buffer and aliquoted 0.1 micrograms per polystyrene well coated with mannan. After a two hour incubation period at room temperature, unbound mannose binding protein was washed from the surface and cell culture supernatant harvested from log-phase growing hybridoma-like cells was added and incubated as described in Method One. The subsequent addition of anti-mouse immunoglobulin conjugated reagents, addition of substrate and quantitation of antibody was performed as described in Method One.

Method three was also used for antibody screening and employed a solution binding assay format. Rabbit antisera directed toward the mannose binding protein was diluted in 0.05 M carbonate buffer, pH 9.6, and used to coat the surface of polystyrene plates by the addition of fifty microliters of diluted sera and incubated overnight at 4°C. On the following day immune mouse sera or cell culture supernatants collected from microtiter wells exhibiting hybridoma-like growth were incubated in a Tween 20/PBS blocked plate along with 0.1 $\mu$g of mannose binding protein diluted in ELISA buffer. Antibody was incubated with the mannose binding protein for a period of two hours at room temperature and then added to the polystyrene plate to which rabbit antisera directed to mannose binding protein was already attached. Incubation of the antibody-MBP complex with the rabbit antisera took place for two hours at room temperature. Excess unbound complex was removed by extensive washings. A dilution of horseradish peroxidase conjugated rabbit anti-mouse immunoglobulin was dispensed per well and incubated for two hours at room temperature. After exhaustive washings, TMB substrate as described above was added and the colorimetric change quantitated using the Multiskan spectrophotometer. In order to assay samples thought or known to contain mannose binding protein using the three immunoassay methods described above, the same procedures are used, except that a sample thought to contain mannose binding protein is also added to the microtiter plate along with the monoclonal antibody which binds mannose binding protein.

Example 5

Determination of Immunoglobulin Class/Subclass

To determine the immunoglobulin class/subclass of the hybridomas, cell culture supernatant containing antibody was assayed in an ELISA format. Affinity purified goat anti-mouse immunoglobulin (heavy and light chain) (Organon Teknika-Cappel) was diluted 1:800 in 0.05 M sodium carbonate buffer, pH 9.6. 50 $\mu$l of this solution was added per well of a distilled water-rinsed Nunc Immuno Plate 1F and incubated 2 hours at room temperature or overnight at 4°C. Unabsorbed antibody was removed by washing with PBS-Tw20. Fifty microliters of antibody-containing supernatant or diluted myeloma ascites controls were added and incubated for a period of two hours at room temperature. Unbound antibody was removed by extensive washings. Alkaline phosphatase conjugated goat anti-mouse immunoglobulin class and subclass specific reagents (FisherBiotech, Orangeburg, NY) were diluted 1:500 in PBS-Tw20 buffer with 0.2% bovine serum albumin. One hundred microliters of each conjugate was added to a well previously incubated with the anti-mouse Ig and the mouse antibody of interest. The alkaline phosphatase conjugate was incubated for two hours at room temperature and the plates washed four times with PBS-Tw20 followed by four washes of Tris buffered saline (0.05 M Tris HCl, 0.15 M NaCl pH 7.5). Alkaline phosphatase activity was visualized using p-nitrophenyl phosphate (1 mg/ml) diluted in alkaline phosphatase buffer (10 mM diethanolamine containing 0.5 mM MgCl). The product of the reaction was scored by eye or quantitated at 405 nm with a Titertek Multiskan PLUS microtiter plate reader.

Example 6

Analytical Methods

Sodium dodecyl sulfate-polyacrylamide gel electrophoresis (SDS-PAGE) was performed using gels and buffers described by Laemmli, U.K., "Cleavage of structural Proteins During Assembly of the Head of Bacteriophage $T_4$", Nature 227:680-685 (1970). Samples were prepared in a buffer containing $\beta$-mercaptoethanol and SDS, heated and subjected to electrophoresis.

Immunoblotting was performed on purified human mannose binding protein and mammalian cell produced recombinant protein that was electrophoresed by SDS-PAGE and electrophoretically transferred to nitrocellulose sheets according to the method of Towbin, H.T., Staehelin, T. and Gordon, J., "Electrophoretic transfer of protein from polyacrylamide gels to nitrocellulose sheets: Procedure and some applications", Proc. Natl. Acad. Sci. (USA), 76:4350-4354 (1979). The nitrocellulose membrane was blocked with 5% BSA (fraction V) prepared in Tris-buffered saline (0.02 M Tris HCl, 0.5 M NaCl, pH 7.5) for 30-60 minutes at room temperature with agitation. The blocked nitrocellulose membrane was then either cut into strips for incubation with antibody containing supernatant or diluted antiserum or placed into a Miniblotter manifold (Immunetics, Cambridge, MA) and appropriate antibodies added to the channels. Incubation with the first antibody was carried out at room temperature with agitation for 1-2 hours. Several washes with TBS were done to remove unbound antibody. An alkaline phosphatase conjugated affinity purified goat antimouse IgG (heavy and light chains) reagent (Jackson ImmunoResearch Laboratories, Inc., West Grove, PA) was diluted 1:3000 in TBS containing 0.05% Tween 20 and 1% gelatin and added to the blot and incubated 1-2 hours at room temperature with agitation. Again, the blot was extensively washed with TBS before addition of substrate. The sites of enzyme binding were detected by the use of BCIP/NBT (5-bromo-4-chloro-3-indolyl phosphate/nitroblue tetrazolium) substrate supplied in kit form from Kirkegaard and Perry (Gaithersburg, MD).

Example 7

Identification and Characterization of Monoclonal Antibodies

Mice immunized with a preparation of human mannose binding protein had developed significant levels of antibody following several immunizations. Sera from the mice were tested by ELISA for the presence of antibodies recognizing mannose binding protein as well as by immunoblotting. The spleen of a responding animal was removed and fused with the myeloma line as described above. Over 75% of the wells plated produced HAT resistant clones distinguishable by light microscopy. Antibody producing hybridomas (prefixed MBP; subclones denoted by .1-.n, for example, MBP7-2 with subclone MBP7-2.59) were identified by ELISA. Forty clones were initially identified; four clones continued to demonstrate such activity following expansion and subcloning. These antibodies (prefixed MBP; subclones denoted by .1-.n) were characterized by determination of specificity with various available proteins. The antibodies (prefixed MabMBP; antibodies produced by subclones denoted by .1-.n as above) were tested for antibody reactivity to $\beta$-galactosidase ($\beta$-gal), bovine serum albumin (BSA), atrial natriuretic factor, bovine thyroglobulin (BTG) and untreated polystyrene following the same format used for the ELISA described above. Several of the monoclonal antibodies bound to the unrelated proteins and were discarded. The heavy chain isotype and light chain class were determined by ELISA as previously described. This data is listed in Table 1. All of the antibodies presumed to be specific to human mannose binding protein based upon the ELISA specificity data were analyzed for reactivity in immunoblotting analysis and results given in Table 1. Polypeptides corresponding to the expected molecular weights of the monomeric, dimeric and trimeric forms of mannose binding protein were visualized with the monoclonal antibodies when the mannose binding protein preparation was electrophoresed in an SDS containing gel and transferred under denaturing conditions. The molecular weight of this protein is in agreement with the expected molecular weight of mannose binding protein. Since this technique involves denaturation of the antigen, recognition by the monoclonal antibodies suggests that the epitopes recognized are either denatured epitopes or denaturation-resistant epitopes. Three antibodies, MBP7-2, MBP8-11 and MBP13-86, are able to recognize the mannose binding protein in solution as well as when the antigen is bound to a plastic support. Inhibition assays were performed in which increasing concentrations of mannose binding protein were added to antibody solutions, antigen-antibody interactions were allowed to proceed and the amount of unbound antibody was determined by binding to mannose binding protein absorbed to plastic. Samples of supernatant-containing antibody were incubated with dilutions of human mannose binding protein (0.0 to 1.2 micrograms) for a period of two hours at room temperature in a polystyrene plate whose protein binding sites had been blocked with the Tween 20/PBS washing buffer. Preincubated samples were then added to a polystyrene plate to which mannose binding protein diluted in carbonate buffer as previously described had been added. Antibody unbound to the soluble mannose binding protein but bound to the plastic-immobilized mannose binding protein was detected using a peroxidase conjugate rabbit anti-mouse immunoglobulin reagent as previously described.

Bound conjugate was quantitated by the addition of TMB substrate and spectrophotometric data recorded. A sample of the results from this experiment are shown in Figure 1. Binding inhibition to plastic-bound mannose binding protein is dose-dependent. Unrelated mouse immunoglobulins do not demonstrate this pattern nor does antibody MBP13-86. These results indicated that the monoclonal antibodies of the present invention can be used in a competitive EIA format.

In order to determine the nature of the epitope recognized by the individual mannose binding protein monoclonal antibodies, an additive binding assay was performed that would identify those monoclonal antibodies binding to the same or spatially close determinants. Based upon these results, it is suggested that monoclonal antibodies MBP7-2 and MBP8-11 recognize epitopes on the molecule so spaced as to allow the simultaneous binding of both antibodies, and therefore these antibodies can be used in a sandwich immunoassay format.

Table 1

CHARACTERIZATION OF MONOCLONAL ANTIBODIES TO MANNOSE BINDING PROTEIN

A. SPECIFICITY AND ISOTYPE DETERMINATION

| Monoclonal Antibody | Isotype | Specificity | | | | | |
|---|---|---|---|---|---|---|---|
| | | Human MBP[1] | BSA | Transferrin | a2macro[3] | β-gal[4] | BTG[5] |
| MabMBP7-2.59 | k,u | + | - | - | - | - | - |
| MabMBP8-11.18 | k,g1 | + | - | - | - | - | - |
| MabMBP8-69 | k,g1 | + | - | - | - | - | - |
| MabMBP13-86 | k,u,g1 | + | - | - | - | - | - |

B. RELATIVE BINDING ACTIVITY

| Monoclonal Antibody | Solution Binding | | Western blot | | ELISA | |
|---|---|---|---|---|---|---|
| | rMBP[2] | Human MBP[1] | Human MBP[1] | rMBP[2] | rMBP[2] | Human MBP[1] |
| MabMBP7-2.59 | + | + | + | + | + | + |
| MabMBP8-11.18 | - | + | - | - | - | + |
| MabMBP8-69 | - | - | - | - | - | + |
| MabMBP13-86 | + | + | + | + | + | + |

[1] Human MBP = Mannose binding protein purified from human sera.
[2] rMBP = Recombinantly produced human mannose binding protein.
[3] a2macro = α-2-macroglobulin
[4] β-gal = β-galactosidase
[5] BTG = bovine thyroglobulin

## Claims

1. A hybrid cell line that produces a monoclonal antibody which binds mannose binding protein.

2. The hybrid cell line according to Claim 1 designated MBP7-2, MBP8-11, MBP8-69 or MBP13-86, or subclones derived therefrom, wherein said hybrid cell lines are obtained by immunization of BALB/c mice with human mannose binding protein, fusion of spleen cells with myeloma cells and culturing the fused cells in HY medium supplemented with hypoxanthine, aminopterin and thymidine.

3. The hybrid cell line with the identifying characteristics of the hybrid cell line according to Claims 1 or 2.

4. The hybrid cell line according to any one of Claims 1 to 3, which is MBP7-2.59 (ATCC HB 10433).

5. A monoclonal antibody secreted by the hybrid cell line according to any one of Claims 1 to 4.

6. The monoclonal antibody according to Claim 5 selected from the group consisting of IgG or IgM.

7. The monoclonal antibody according to Claims 5 or 6 which is a murine monoclonal antibody.

8. The monoclonal antibody according to any one of Claims 5 to 7 designated MabMBP7-2, MabMBP8-11, MabMBP8-69, or MAbMBP13-86, wherein said monoclonal antibodies exhibit no crossreactivity to BSA, transferrin, a2macro, $\beta$-gal or BTG.

9. The monoclonal antibody with the identifying characteristics of the monoclonal antibody according to Claim 8.

10. The monoclonal antibody according to any one of Claims 5 to 9 which has been substantially purified.

11. The monoclonal antibody according to any one of Claims 5 to 10 which has been derivatized.

12. The monoclonal antibody according to any one of Claims 5 to 11 which has been labelled with a radioisotope.

13. The monoclonal antibody according to any one of Claims 5 to 11 which has been conjugated to an enzyme.

14. The monoclonal antibody according to any one of Claims 5 to 11 which has been conjugated to a toxin.

15. The monoclonal antibody according to any one of Claims 5 to 11 which has been conjugated to an enzyme inhibitor.

16. An immunoassay method for detecting the presence of mannose binding protein in a sample comprising:
    (a) incubating the sample with a monoclonal antibody or fragment thereof which binds to the mannose binding protein; and
    (b) detecting the presence of immune complexes formed by the mannose binding protein and the monoclonal antibody or fragment thereof.

17. An immunoassay method for quantitatively determining the amount of mannose binding protein in a sample comprising:
    (a) incubating the sample with a monoclonal antibody or fragment thereof which binds to the mannose binding protein;
    (b) determining the amount of immune complexes formed by the mannose binding protein and the monoclonal antibody or fragment thereof; and
    (c) correlating the amount of immune complexes formed with the amount of mannose binding protein present in the sample.

18. The immunoassay method according to Claims 16 or 17 which is a radioimmunoassay, an enzyme immunoassay or an immunoblotting assay.

19. The monoclonal antibody according to Claim 14 or 15 for treating an infection caused by a pathogen comprising administering to a mammalian host a therapeutically effective amount of said monoclonal antibody.

12

20. A composition for treating an infection caused by a pathogen comprising a therapeutically effective amount of the monoclonal antibody according to Claims 14 or 15 and a pharmaceutically acceptable carrier thereof.

21. Use of the monoclonal antibody according to Claims 14 or 15 for the preparation of a medicament for treating pathogen-caused infections in a mammalian host.

22. A kit comprising at least one monoclonal antibody according to any one of Claims 5 to 15.

FIG. 1
INHIBITION OF ANTIBODY BINDING TO
BOUND MBP BY SOLUBLE MBP

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | WO-A-8 901 519 (CHILDREN'S HOSPITAL CORPORATION)<br><br>* page 12, line 16 - line 21; claims 28-31 *<br>--- | 1-11, 16-18,22 | C12N5/20<br>C12P21/08<br>G01N33/577<br>G01N33/68<br>A61K47/48 |
| A | THE JOURNAL OF IMMUNOLOGY<br>vol. 144, no. 6, 15 March 1990, BALTIMORE, USA<br>pages 2287 - 2294;<br>J. LU ET AL.: 'Binding of the pentamer/hexamer forms of mannan-binding protein to zymosan activates the proenzyme C1r2C1s2 complex, of the classical pathway of complement, without involvement of C1q.'<br>* page 2288, right column, line 59 - page 2289, left column, line 4 *<br>--- | 5-10, 16-18,22 | |
| A | JOURNAL OF BIOCHEMISTRY<br>vol. 101, no. 1, 1987, TOKYO, JAPAN<br>pages 135 - 144;<br>S. OKA ET AL.: 'Primary structure of rat liver mannan-binding protein deduced from its cDNA sequence.'<br>* page 137, left column, line 5 - line 33 *<br>--- | 5-10, 16-18,22 | |
| A | BIOCHIMICA ET BIOPHYSICA ACTA<br>vol. 883, no. 2, 4 September 1986, AMSTERDAM, NL<br>pages 197 - 206;<br>J. SUMMERFIELD ET AL.: 'Mannose-binding proteins in human serum: Identification of mannose-specific immunoglobulins and a calcium-dependent lectin, of broader carbohydrate specificity, secreted by hepatocytes.'<br>* abstract *<br>--- | 5-10, 16-18,22 | **TECHNICAL FIELDS SEARCHED (Int. Cl.5 )**<br><br>C07K<br>G01N<br>C12N<br>C12P |
| P,X | WO-A-9 106 010 (INSTITUTE OF CHILD HEALTH)<br><br>* claims *<br><br>----- | 1-11,13, 16-18,22 | |

The present search report has been drawn up for all claims

| Place of search | Date of completion of the search | Examiner |
|---|---|---|
| THE HAGUE | 27 MARCH 1992 | NOOIJ F.J.M. |